# EUROPEAN PATENT SPECIFICATION

(11) **EP 1 778 191 B1**
(45) Date of publication and mention of the grant of the patent: **08.06.2016**
(21) Application number: 05808427.8
(22) Date of filing: 11.08.2005
(51) Int. Cl.: A61K 9/14, A61F 13/00, A61F 2/00

(54) **BIODEGRADABLE DIBLOCK COPOLYMERS HAVING REVERSE THERMAL GELATION PROPERTIES AND METHODS OF USE THEREOF**
BIOLOGISCH ABBAUBARE DIBLOCKCOPOLYMERE MIT EIGENSCHAFTEN DER REVERSEN THERMISCHE GELIERUNG UND VERWENDUNGSVERFAHREN DAFÜR
COPOLYMERES DIBLOCS BIODEGRADABLES A PROPRIETES DE GELIFICATION THERMIQUE INVERSEE ET LEURS PROCEDES D'UTILISATION

(30) Priority: 16.08.2004 US 919603
(43) Date of publication of application: 02.05.2007
(73) Proprietor: Protherics Salt Lake City, Inc., West Valley City, UT 84120 (US)
(72) Inventor: JO. Seongbong, Salk Lake City, UT 84124 (US); PIAO, Ai-Zhi, Salt Lake City, UT 84111 (US)
(74) Representative: BTG plc Intellectual Property Group
(86) International application number: PCT/US2005/028710
(87) International publication number: WO 2006/023388

(56) References cited:
- US-A1- 2004 001 872
- US-B2- 6 592 899

## Description

### BACKGROUND OF THE INVENTION

### Field of the Invention

The present invention relates generally to an aqueous biodegradable polymeric drug delivery composition possessing reverse thermal gelation properties. Further described are water soluble, low molecular weight, thermosensitive, biodegradable diblock copolymers having a high weight percentage (about 61 to 85 wt%) of biodegradable hydrophobic block(s), and their use for the parenteral, ocular, topical, transdermal, vaginal, buccal, transmucosal, pulmonary, transurethral, rectal, nasal, oral, or aural administration of drugs. Further described are thermosensitive biodegradable diblock copolymers based on biodegradable polyester, polyester amide, polyether ester, polyurethane, polyester urethane, polycarbonate, or polyester carbonates and low molecular weight (less than 5000 daltons) polyethylene glycol(PEG) blocks. The system is based on the discovery that only a select subset of such diblock copolymers with relatively low molecular weights (less than 15000 daltons) and relatively high hydrophobic block polymer content(more than 61%) exist as solutions at, or about, 5°C to 34°C in water but, when the temperature is raised to about body temperature (typically 37°C for humans), they spontaneously interact to form semisolid hydrogels (i.e., gels) that contain a high percentage of water entrapped within the gel network and yet are substantially insoluble in water.

### Related Art

Recently, many peptide/protein drugs, effective for a variety of therapeutic applications, have become commercially available through advances in recombinant DNA and other technologies. However, polypeptides or proteins, with their high molecular weight, degradation by gastrointestinal tract enzymes, and short half-life in the body are limited to parenteral administration by such routes as intravenous, intramuscular or subcutaneous injection. Many peptide drugs are of limited solubility and/or stability in conventional liquid carriers and are therefore difficult to formulate and administer. Also, in many cases, numerous administrations are required to get the expected therapeutic effect for an extended period of time. Long-term, controlled delivery of such polypeptides or proteins is essential to provide for the practical application of these medications and to utilize advanced biotechnological derived drugs. Another problem is patient compliance. It is often difficult to get a patient to follow a prescribed dosage regimen, particularly when the prescription is for a chronic disorder and the drug has acute side effects. Therefore, it would be highly desirable to provide a system for the delivery of polypeptide and protein drugs at a controlled rate over a sustained period of time without the above-mentioned problems in order to optimize the therapeutic efficacy, minimize the side effects and toxicity, and thereby increase the efficacy and increase patient compliance.

Drug loaded polymeric devices and dosage forms have been investigated for long term therapeutic treatment of different diseases. An important property of the polymer is biodegradability, meaning that the polymer can break down or degrade within the body to nontoxic components either concomitant with release of the drug, or, after all of the drug has been released. Furthermore, techniques, procedures, solvents and other additives used to fabricate the device and load the drug should result in dosage forms that are safe for the patient, minimize irritation to surrounding tissue, and be a compatible medium for the drug.

Many biodegradable implantable controlled release devices are fabricated from solid polymers such as polyglycolic acid, polylactic acid, or copolymers of glycolic and lactic acid. Due to the hydrophobic properties of these polymers, drug loading and device fabrication using these materials requires use of organic solvents, for example, methylene chloride, chloroform, acetic acid or dimethyl formamide. Due to the toxic nature of some solvents, extensive drying to remove excess solvent is generally required after this process. In most cases the final polymeric device is fabricated in a distinct solid shape (e.g., sphere, slab or rod) requiring an implantation procedure which often results in trauma to tissue.

Currently there are few synthetic or natural polymeric materials which can be used for the controlled delivery of drugs, including peptide and protein drugs, because of strict regulatory compliance requirements, such as biocompatibility, having a clearly defined degradation pathway, and safety of the degradation products. The most widely investigated and advanced biodegradable polymers in regard to available toxicological and clinical data are the aliphatic poly(α-hydroxy acids), such as poly(D,L- or L- lactic acid) (PLA) and poly(glycolic acid) (PGA) and their copolymers (PLGA). These polymers are commercially available and are presently being used as bioresorbable sutures. An FDA-approved system for controlled release of leuprolide acetate, Lupron DepotJ, is also based on PLGA copolymers. Lupron DepotJ consists of injectable microspheres, which release leuprolide acetate over a prolonged period of time(e.g., about 30 days) for the treatment of prostate cancer. Based on this history of use, PLGA copolymers have been the materials of choice in the initial design of parenteral controlled release drug delivery systems which uses a biodegradable carrier.

Even though there has been some limited success, these polymers have problems associated with their physicochemical properties and methods of fabrication. Hydrophilic macromolecules, such as polypeptides, cannot readily diffuse through hydrophobic matrices or the membranes of polylactides. Drug loading and device fabrication using PLA and PLGA often require use of toxic organic solvents, and the solid dosage form may mechanically induce tissue irritation.

A.S. Sawhney and J.A. Hubbell, J. Biomed. Mat. Res., 24, 1197-1411 (1990), synthesized terpolymers of D,L-lactide, glycolide and ε-caprolactone which degrade rapidly *in vitro.* For example, a terpolymer composition of 60% glycolide, 30% lactide, and 10% ε-caprolactone exhibited a half-life of 17 days. The hydrophilicity of the material was increased by copolymerization with a poloxamer surfactant (Pluronic® F-68). This poloxamer is a block copolymer comprising about 20% by weight of a relatively hydrophobic poly(oxypropylene) block and 80% by weight of a hydrophilic poly(oxyethylene) block. Copolymerization with the poloxamer resulted in a stronger and partly crystalline material which was mechanically stable in water at physiological temperatures (e.g. 37°C). The half-life of this copolymer was slightly increased compared to the base polymer. However, it is known that poloxamer-type surfactants are not biodegradable.

An optimum material for use as an injectable or implantable polymeric drug delivery device should be biodegradable, compatible with hydrophilic or hydrophobic drugs, be fabricated with simple, safe solvents, such as water, and not require additional polymerization or other covalent bond forming reactions following administration.

One system which can be fabricated in aqueous solution is a class of block copolymers referenced above and marketed as PluronicJ®. These copolymers are composed of two different polymer blocks, i.e. hydrophilic poly(oxyethylene) blocks and hydrophobic poly(oxypropylene) blocks, making a triblock of poly(oxyethylene)-poly(oxypropylene)-poly(oxyethylene). The triblock copolymers to form gels in water which exhibit reverse thermal gelation behavior. However, the PluronicJ® system is nonbiodegradable and the gel properties (water soluble gel) and drug release kinetics (very rapid) from those gels have not proven useful and are in need of substantial improvement.

There is a strong need for hydrophilic biodegradable materials which can be used to incorporate water soluble polypeptide drugs in solution. A.S. Sawhney et al., Macromolecules, Vol 26, No. 4, 581-589 (1993) synthesized macromers having a polyethylene glycol central block, extended with oligomers of α-hydroxy acids such as oligo(D,L-lactic acid) or oligo(glycolic acid) and terminated with acrylate groups. Using nontoxic photoinitiators, these macromers can be rapidly polymerized with visible light. Due to the multifunctionality of the macromers, polymerization results in the formation of crosslinked gels. The gels degrade upon hydrolysis of the oligo(α-hydroxy acid) regions into polyethylene glycol, the α-hydroxy acid, and oligo(acrylic acid), and their degradation rates can be tailored by appropriate choice of the oligo(α-hydroxy acid) to be from less than 1 day to up to 4 months. However, in this system, an additional component, a photoinitiator is employed, as well as the need for an additional covalent bond-forming photocrosslinking reaction. Highly variable person-to-person performance would result from this approach due to interperson differences in skin thickness and opacity.

Okada et al., Japanese Patent 2-78629 (1990), synthesized biodegradable block copolymeric materials by trans-esterification of poly(lactic acid) (PLA) or poly(lactic acid)/glycolic acid (PLGA) and polyethylene glycol (PEG). The molecular weight range for PLGA block was 400 to 5,000 and for the PEG block, 200 to 2,000. The mixture was heated at 100°C to 250°C for 1 to 20 hours under a nitrogen atmosphere. The product was miscible with water and formed a hydrogel; however, it precipitated in water above room temperature. In other words, the water solubility and interpolymer chain interactions changed with temperature. This polymer is similar to the polymers described in the Churchill patents discussed below and is utilized as an aqueous suspension or molded into a solid block for implantation. However, there is no indication that this polymer exhibits properties of reverse thermal gelation.

T. Matsuda, ASAIO Journal, M512-M517 (1993) used a biodegradable polymeric gel to deliver a potent peptidyl antiproliferative agent, angiopeptin, to prevent the myointimal hyperplasia that occurs when a diseased vessel is replaced with an artificial graft or is treated by an intravascular device. A highly viscous liquid of a block copolymer composed of poly(lactic acid) and polyethylene glycol (PLA-PEG) block segments was used as an *in situ* coatable drug carrier. The materials were supplied by Taki Chemical Co., Ltd., Hyogo, Japan. A prolonged slow release of angiopeptin from the polymer gel, consisting of 0.5g PLA-PEG and 0.5 mg angiopeptin, was observed *in vitro* over a few weeks when the gel was kept in a buffer solution maintained at 37°C. No early burst release of angiopeptin was observed. Based on these results, a local sustained angiopeptin release from the biodegradable polymeric gel that was coated onto the injured vessel *in vivo* was theorized to be effective.

L. Martini et al., J. Chem. Soc., Faraday Trans., 90(13), 1961-1966 (1994) synthesized very low molecular weight ABA type triblock copolymers by incorporating hydrophobic poly(ε-caprolactone), which is known to be subject to degradation *in vivo* by hydrolytic chain scission involving the ester linkages, and they reported the solution properties of the PCL-PEG-PCL block copolymers. Clouding was observed when an aqueous solution of the block copolymers was slowly heated. The temperatures when 2 wt% aqueous solutions of the copolymers turn cloudy were 65°C and 55°C for PCL-PEG-PCL (450:4000:450) and PCL-PEG-PCL (680:4000:680), respectively. Reversible gelation on cooling of the solutions of PCL-PEG-PCL (680:4000:680) was observed at critical concentrations and temperatures ranging from 13% at 25°C to 30% at 80°C. No lower gel/sol transition was observed on further cooling the solutions to °C . The *in vitro* degradation rate of PCL-PEG-PCL (680:4000:680) was very slow. Only about a 20% decrease in molar mass (from GPC) was observed over a 16 week period. Such slow degradation is insufficient for a practical drug delivery vehicle.

Churchill et al., U.S. Patents 4,526,938 and 4,745,160 show copolymers that are either self-dispersible or can be made self-dispersible in aqueous solutions. These copolymers are ABA triblock or AB diblock copolymers composed of hydrophobic A-blocks, such as polylactide (PLA) or poly(lactide-co-glycolide)(PLGA), that are functional with molecular weight of less than 5000. Furthermore, there is no exemplification of ABA type polymers other than high molecular weigh without the use of organic solvents and hydrophilic B-bloclcs, such as polyethylene glycol (PEG) or polyvinyl pyrrolidone. The copolymers described may be self-dispersible in water without the use of organic solvents, these polymers must contain more than 50% by weight of hydrophilic (B-block) components as compared to hydrophobic (A block) components, or, are copolymers where the hydrophobic component (A block) has an average molecular weight of less than 5,000. Although polymers having an average molecular weight as low as 1000 are mentioned, there is no indication that without the use of organic solvents these block copolymers are soluble in aqueous solutions at any temperature, nor is there any indication that the drug/block copolymers can be administered as a solution. Rather, administration as a colloidal suspension of the polymer, or, drug/polymer dispersions that are freeze dried into a powder and processed by compression moulding to form a solid suitable for use as an implantable depot formulation are described. Aqueous drug/polymer suspensions or dispersions are two phase systems wherein the dispersed polymer phase is suspended in the continuous aqueous phase. Such dispersions are not suitable for use in situations where sterile filtering processes are required to remove bacterial or other toxic particulates, as any such process would also remove the drug/polymer particles and result in sub-therapeutic doses. ABA-type block copolymers that are water soluble and that gel thermally are not included nor taught in the Churchill, et al., patents.

Jeong et al., Nature, 388, 860,1997, discloses the use of high molecular weight PEG in di- and triblock copolymers to make a biodegradable thermosensitive hydrogel. However, the hydrophobic block (PLGA) of Jeong's block copolymer is less than 60 weight %. In addition, the block copolymer solution undergoes gelation during cooling.

Rathi et al., US patents 6,117,949; 6,201,072; 6,004,573; and 5,702,717 disclose biodegradable triblock copolymers that exhibit reverse thermal gelation behavior, namely, exist as a liquid solution at low temperatures, then reversibly form gels at physiologically relevant temperatures, and provide good drug release characteristics. Further, the biodegradable ABA- or BAB=type block copolymer having an average molecular weight of between about 2000 and 4990, consisting of about 51 to 83% by weight of an hydrophobic A polymer block comprising a biodegradable polyester and about 17 to 49% by weight of a hydrophilic B polymer block consisting of polyethylene glycol(PEG).

Jeong et al, J. Polym. Science Part A, 37, 751, 1999, Choi et al. J. Polym. Science Part A, 37, 2207, 1999, discloses series of di- and triblock copolymers wherein the hydrophilic block, MeO-PEG, has a molecular weight higher than 850 and the percentage of the hydrophobic block (PLGA) is less than 60 weight %. In addition, the block copolymer solution also undergoes gelation by cooling the temperature. Jeong et al., US 2002/0173586, discloses a branched polymer showing thermosensitive gelation properties.

U.S. Patent Application Publication No. 2004/001872 discloses di- and tri-block polymers which are free flowing liquids at body temperature, e.g., between 35-42°C.

U.S. Patent No. 6,592,899 discloses compositions that remain soluble and free flowing at physiologically relevant temperatures and are not compositions having reverse thermal gelation properties.

Although there are triblock copolymers that possess reverse thermal gelation properties, the diblock copolymer described possess release kinetics of proteins and peptides in a controlled manner compared to previously developed gels. One big advantage of the diblock copolymers described is that their low molecular weight gives more flexibility in the design of small hydrophilic or macromolecular injectable drug delivery systems. Thus, the block copolymer concentration in a drug formulation can be easily increased to form tight networks for sustained release of entrapped drugs.

### SUMMARY OF THE INVENTION

The present invention provides biodegradable diblock copolymers which have reverse thermal gelation properties. The diblock copolymers have more preferable release properties for the delivery of proteins and peptides than previously known hydrogels. General belief is that physical gels based on various physical interactions have been of very limited use for delivery of hydrophilic macromolecules mainly because of low polymer to water ratio resulting in loose networks. Compared to previously known hydrogels, the diblock copolymers described have lower molecular weights than triblock copolymers. Thus, the block copolymer concentrations can be easily increased for an application as protein or peptide delivery systems with the advantageous formation of tight networks, The diblock copolymers described also possess reverse thermal gelation properties. Previous publications have also demonstrated thermal gelation of diblock copolymers. However, previous work showed that aqueous solutions of diblock copolymers with low hydrophobic block content(less than 61%) underwent gelation by lowering the temperature (cooling). This phenomenon has been scientifically described as "Upper Critical Solution Temperature" (UCST). However, the novel diblock copolymers described have reverse thermal gelation properties which are based on a rise in temperature. This phenomenon has been scientifically described as "Lower Critical Solution Temperature" (LCST). The latter phenomenon is more preferable for formulation of injectable drug delivery systems.

Therefore, low molecular weight diblock copolymer drug delivery systems that are biodegradable, exhibit reverse thermal gelation behavior, namely, exist as a liquid solution at low temperatures, reversibly form gels at physiologically relevant temperatures, and provide good drug release characteristics are described.

The present invention discloses an aqueous biodegradable polymeric drug delivery composition possessing reverse thermal gelation properties comprised of an aqueous phase having uniformly contained therein: (a) an effective amount of a drug; and (b) a biodegradable AB type di block copolymer comprising: i) 61 to 85 % by weight of a biodegradable, hydrophobic A block which is a member selected from the group consisting of biodegradable polyesters, biodegradable polyester amides, biodegradable polyether esters, biodegradable polyurethanes, biodegradable polyester urethanes, biodegradable polycarbonates and polyester carbonates; and ii) 15 to 39 % by weight of a biocompatible, hydrophilic B block comprising a mono functional polyethylene glycol(PEG), wherein, each hydrophobic A block has a number average molecular weight of between 1000 Daltons and 4000 Daltons and each hydrophilic B block has a number average molecular weight of between 500 Daltons and 800 Daltons; and wherein said di block copolymer has a number average molecular weight within a range of 450 Daltons to 15000 Daltons and wherein the copolymer possesses reverse thermal gelation properties such that it exists as a solution at about 5-34°C in water, but at 37°C, the copolymers spontaneously interact to form a semisolid hydrogel.

Preferably, the biodegradable, hydrophobic A block is synthesized from monomers selected from the group consisting of D,L-lactide, D-lactide, L-lactide, D,L-lactic acid, D-lactic acid, L-lactic acid, glycolide, glycolic acid, p-dioxanone, trimethylene carbonate, ε-caprolactone, ε-hydroxy hexonoic acid, γ-butyrolactone, γ-hydroxy butyric acid, δ-valerolactone, δ-hydroxy valeric acid, hydrooxybutyric acids, malic acid, and copolymers thereof.

Preferably, the di block polymer content of said composition is between 3 and 50% by weight and the drug content of said composition is between 0.01 and 20% by weight.

The present invention discloses a method for enhancing the solubility of a drug, comprising uniformly admixing an effective amount of said drug in an aqueous biodegradable polymeric drug delivery composition possessing reverse thermal gelation properties, said aqueous composition being comprised of an aqueous phase having uniformly contained therein a biodegradable AB type di block copolymer comprising: i) 61 to 85 % by weight of a biodegradable, hydrophobic A block which is a member selected from the group consisting of biodegradable polyesters, biodegradable polyester amides, biodegradable polyether esters, biodegradable polyurethanes, biodegradable polyester urethanes, biodegradable polycarbonates and polyester carbonates; and ii) 15 to 39 % by weight of a biocompatible, hydrophilic B block comprising a mono functional polyethylene glycol(PEG), wherein, each hydrophobic A block has a number average molecular weight of between 1000 Daltons and 4000 Daltons and each hydrophilic B block has a number average molecular weight of between 500 Daltons and 800 Daltons; and wherein the copolymer possesses reverse thermal gelation properties such that it exists as a solution at about 5-34°C in water, but at 37°C, the copolymers spontaneously interact to form a semisolid hydrogel.

The present invention discloses a method for enhancing the stability of a drug comprising uniformly admixing an effective amount of said drug in an aqueous biodegradable polymeric drug delivery composition possessing reverse thermal gelation properties said aqueous composition being comprised of an aqueous phase having uniformly contained therein a biodegradable AB type di block copolymer comprising: i) 61 to 85 % by weight of a biodegradable, hydrophobic A block which is a member selected from the group consisting of biodegradable polyesters, biodegradable polyester amides, biodegradable polyether esters, biodegradable polyurethanes, biodegradable polyester urethanes, biodegradable polycarbonates and polyester carbonates; and ii) 15 to 39 % by weight of a biocompatible, hydrophilic B block comprising a mono functional polyethylene glycol(PEG), wherein, each hydrophobic A block has a number average molecular weight of between 1000 Daltons and 4000 Daltons and each hydrophilic B block has a number average molecular weight of between 500 Daltons and 800 Daltons; and wherein the copolymer possesses reverse thermal gelation properties such that it exists as a solution at about 5-34°C in water, but at 37°C, the copolymers spontaneously interact to form a semisolid hydrogel.

Preferably, the biodegradable, hydrophobic A block is synthesized from monomers selected from the group consisting of D,L-lactide, D-lactide, L-lactide, D,L-lactic acid, D=lactic acid, L-lactic acid, glycolide, glycolic acid, p-dioxanone, trimethylene carbonate, ε-caprolactone, ε-hydroxy hexonoic acid, γ-butyrolactone, γ-hydroxy butyric acid, δ-valerolactone, δ-hydroxy valeric acid, hydrooxybutyric acids, malic acid, and copolymers thereof.

More preferably, the di block polymer content of said composition is between 3 and 50% by weight and the drug content of said composition is between 0.01 and 20% by weight.

The present invention also provides a drug delivery system for the parenteral administration of hydrophilic and hydrophobic drugs, peptide and protein drugs, hormones, genes/nucleic acids, oligonucleotides and anticancer agents.

The present invention also provides a method for the parenteral administration of drugs in a biodegradable polymeric matrix resulting in the formation of a gel depot within the body, from which the drugs are released at a controlled rate.

A biodegradable AB type diblock copolymers having an average molecular weight of between 450 and 15000, consisting of 61 to 85% by weight of a hydrophobic A polymer block which is a member selected from the group consisting of biodegradable polyesters, biodegradable polyester amides, biodegradable polyether esters, biodegradable polyurethanes, biodegradable polyester urethanes, biodegradable polycarbonates and polyester carbonates and 15 to 39% by weight of a hydrophilic B polymer block consisting of polyethylene glycol(PEG) is described.

The biodegradable polyester is synthesized from monomers selected from the group consisting of D,L-lactide, D-lactide, L-lactide, D,L-lactic acid, D-lactic acid, L-lactic acid, glycolide, glycolic acid, p-dioxanone, ε-caprolactone, δ-hydroxy hexonoic acid, γ-butyrolactone, γ-hydroxy butyric acid, δ-valerotactone, δ-hydroxy valeric acid, hydrooxybutyric acids, malic acid, trimethylene carbonate, and copolymers thereof. The biodegradable polyester is synthesized from monomers selected from the group consisting of D,L-lactide, D-lactide, L-lactide, D,L-lactic acid, D-lactic acid, L-lactic acid, glycolide, glycolic acid, ε-caprolactone, ε-hydroxy hexonoic acid, and copolymers thereof.

The biodegradable polyester is synthesized from monomers selected from the group consisting of D,L-lactide, D-lactide, L-lactide, D,L-lactic acid, D-lactic acid, L-lactic acid, glycolide, glycolic acid, and copolymers thereof.

Polyethylene glycol (PEG) is also sometimes referred to as poly(ethylene oxide) (PEO) or poly(oxyethylene) and the terms can be used interchangeably for the purposes of this invention. The hydrophilic block described is a PEG having a number average molecular weight between 50 and 5000 daltons, between 350 and 2000 daltons and further may be 500 and 800 daltons. Preferably, the hydrophobic block described has a number average molecular weight between 500 and 5000 daltons, or between 1000 and 4000 daltons.

Additional features and advantages of the invention will be apparent from the detailed description which follows, taken in conjunction with the accompanying drawings, which together illustrate, by way of example, features of the invention.

### BRIEF DESCRIPTION OF THE DRAWINGS

The above and other features and advantages of the invention will become apparent from a consideration of the following detailed description presented in connection with the accompanying drawings in which:
FIG. 1 is a reaction scheme illustrating diblock copolymer synthesis by ring opening polymerization
FIG. 2 is a reaction scheme illustrating diblock copolymer synthesis by condensation polymerization.
FIG. 3 is a phase diagram illustrating the gelation behavior of aqueous solutions of a PLG-PEG diblock copolymer, studied at different concentrations and temperatures.
FIG. 4 is a graph illustrating the continuous release of FITC Dextrin 40000 over a sustained period of time from a PLG-PEG diblock copolymer thermal gel.
FIG. 5-a compares in vitro release of FITC dextran 4,000 from AB diblock copolymer thermal gels at 30 % and 40% polymer concentrations.

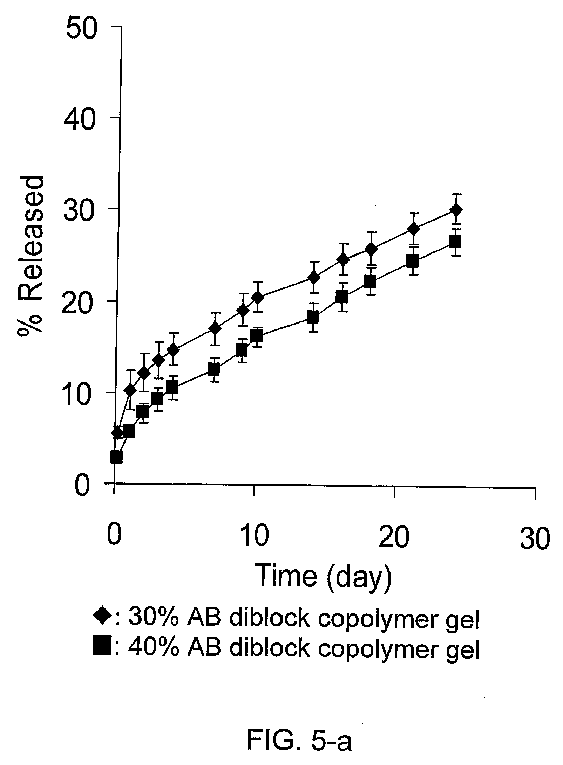

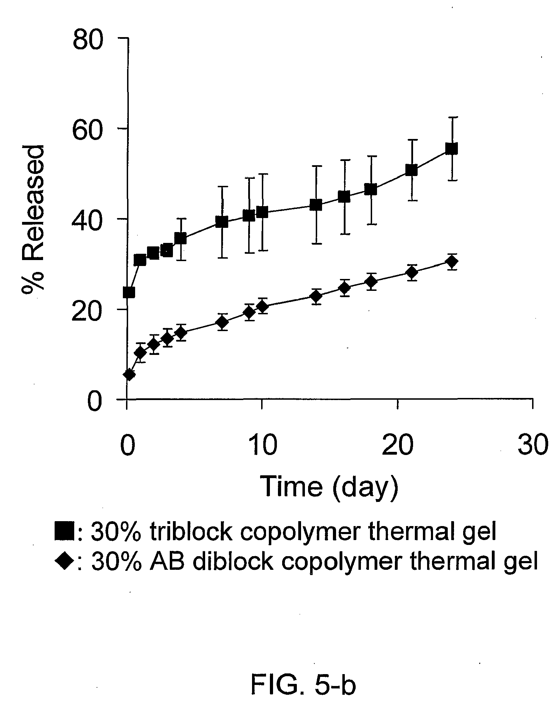

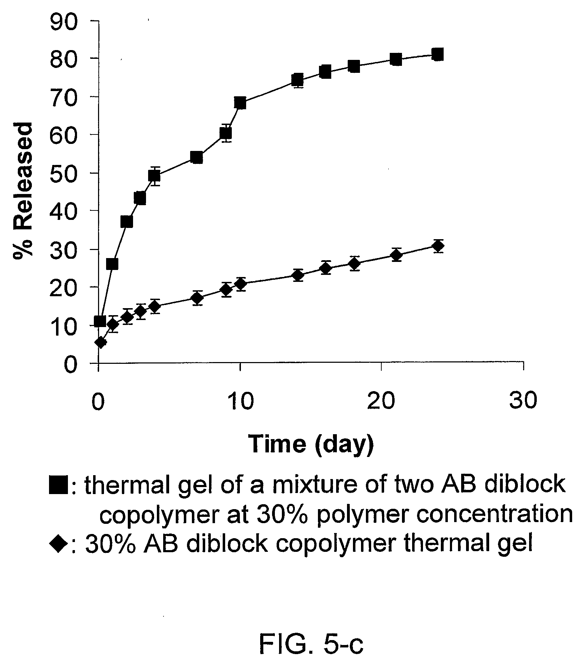

FIG 5-b compares in vitro release of FITC dextran 4,000 from AB diblock copolymers and triblock copolymer thermal gels at a 30% polymer concentration.
FIG 5-c compares in vitro release of FITC dextran 4,000 from AB diblock copolymers and a mixture of two diblock copolymer thermal gels at a 23% polymer concentration.

### DETAILED DESCRIPTION

As used herein the following terms shall have the assigned meanings:
"Parenteral" shall mean intramuscular, intraperitoneal, intra-abdominal, subcutaneous, and, to the extent feasible, intravenous and intraarterial.
"Gelation temperature" means the temperature at which the biodegradable block copolymer undergoes reverse thermal gelation, i.e. the temperature below which the block copolymer is soluble in water and above which the block copolymer undergoes phase transition to increase in viscosity or to form a semi-solid gel.

The terms "gelation temperature" and "reverse thermal gelation temperature" or the like shall be used interchangeably in referring to the gelation temperature.

"Polymer solution," "aqueous solution" and the like, when used in reference to a biodegradable block copolymer contained in such solution, shall mean a water based solution having such block copolymer dissolved therein at a functional concentration, and maintained at a temperature below the gelation temperature of the block copolymer.

"Reverse thermal gelation" is the phenomena whereby a solution of a block copolymer spontaneously increases in viscosity, and in many instances transforms into a semisolid gel, as the temperature of the solution is increased above the gelation temperature of the copolymer. For the purposes of the invention, the term "gel" includes both the semisolid gel state and the high viscosity state that exists above the gelation temperature. When cooled to below the gelation temperature, the gel spontaneously reverses to reform the lower viscosity solution. This cycling between the solution and the gel may be repeated *ad infinitum* because the sol/gel transition does not involve any change in the chemical composition of the polymer system. All interactions to create the gel are physical in nature and do not involve the formation or breaking of covalent bonds.

"Drug delivery liquid" or "drug delivery liquid having reverse thermal gelation properties" shall mean a polymer solution that contains drug (the drug *per se* can either be dissolved or colloidal) suitable for administration to a warm-blooded animal which forms a gelled drug depot when the temperature is raised to or above the gelation temperature of the block copolymer.

"Depot" means a drug delivery liquid following administration to a warm-blooded animal which has formed a gel upon the temperature being raised to or above the gelation temperature.

"Gel" means the semi-solid phase that spontaneously occurs as the temperature of the "polymer solution" or "drug delivery liquid" is raised to or above the gelation temperature of the block copolymer.

"Aqueous polymer composition" means either a drug delivery liquid or a gel comprised of the water phase having uniformly contained therein a drug and the biodegradable block copolymer. At temperatures below the gelation temperature the copolymer may be soluble in the water phase and the composition will be a solution. At temperatures at or above the gelation temperature the copolymer will solidify to form a gel with the water phase, and the composition will be a gel or semi-solid.

`Biodegradable" means that the block copolymer can chemically break down or degrade within the body to form nontoxic components. The rate of degradation can be the same or different from the rate of drug release.

"Drug" shall mean any organic or inorganic compound or substance having bioactivity and adapted or used for a therapeutic purpose. Proteins, hormones, anticancer agents, oligonucleotides, DNA, RNA and gene therapies are included under the broader definition of drug.

"Peptide," "polypeptide," "oligopeptide" and "protein" shall be used interchangeably when referring to peptide or protein drugs and shall not be limited as to any particular molecular weight, peptide sequence or length, field of bioactivity or therapeutic use unless specifically stated.

"Poly(lactide-co-glycolide)" or "PLGA" shall mean a copolymer derived from the condensation copolymerization of lactic acid and glycolic acid, or, by the ring opening polymerization of α-hydroxy acid precursors, such as lactide or glycolide. The terms "lactide," "lactate," "glycolide" and "glycolate" are used interchangeably.

"Poly(glycolide)" or "PLG" shall mean a polymer derived from the condensation of glycolic acid or by the ring opening polymerization of glycolide.

"Biodegradable polyesters" refers to any biodegradable polyesters, which are synthesized from monomers selected from the group consisting of D,L-lactide, D-lactide, L-lactide, D,L-lactic acid, D-lactic acid, L-lactic acid, glycolide, glycolic acid, p-dioxanone, trimethylene carbonate, ε-caprolactone, ε-hydroxy hexonoic acid, γ-butyrolactone, γ-hydroxy butyric acid, δ-valerolactone, δ-hydroxy valeric acid, hydrooxybutyric acids, malic acid, and copolymers thereof.

Therefore, the present invention is based on the discovery of AB type diblock copolymers, where the A-block comprises a hydrophobic biodegradable polyester, and the B-block is a polyethylene glycol (PEG) having a molecular weight lower than 5000 daltons, having a hydrophobic content of between 61 to 85% by weight and an overall block copolymer molecular weight within a range of 450 any 15000 daltons, and which exhibits water solubility at low temperature and undergoes reversible thermal gelation at mammalian physiological body temperatures. With such high hydrophobic content, it is unexpected that such diblock copolymers would be water soluble. It is generally taught that any polymer having a hydrophobic content in excess of 50% by weight is substantially insoluble in water and can only be made appreciably soluble in aqueous systems, if at all, when a certain amount of an organic co-solvent has been added.

The diblock copolymers that have utility as described meet the criteria summarized in Table 1, namely having a compositional make-up within the indicated ranges that result in diblock copolymers demonstrating the desired reverse thermal gelling behavior. For purposes of disclosing molecular weight parameters, all reported molecular weight values are based on measurements by NMR or GPC (gel permeation chromatography) analytical techniques. The reported average molecular weights and number average molecular weights were determined by GPC and NMR respectively (when GPC is used to determine molecular weight of diblock copolymers, the MW may be higher). The reported lactide/glycolide ratio was calculated from NMR data. GPC analysis was performed on a Styragel HR-3 column calibrated with PEG using RI detection and chloroform as the eluent, or on a combination of Phenogel, a mixed bed, and Phenogel, 500 Å columns calibrated with PEG using RI detection and tetrahydrofuran as the eluent. NMR spectra were taken in CDCl₃ on a Bruker 200 MHz instrument.

**Table 1**

| | |
|---|---|
| Total weight average molecular weight: | < 15000 |
| PEG content: | 15 to 39% by weight |
| Total polyester content: | 61 to 85% by weight |
| Lactate content: | 20 to 100 mole percent |
| Glycolate content: | 0 to 80 mole percent |
| Behavior: | water soluble below the gelation temperature; gels above the gelation temperature |

The biodegradable, hydrophobic A polymer block comprises a polyester synthesized from monomers selected from the group consisting of D,L-lactide, D-lactide, L-lactide, D,L-lactic acid, D-lactic acid, L-lactic acid, glycolide, glycolic acid, p-dioxanone, trimethylene carbonate, ε-caprolactone, ε-hydroxy hexonoic acid, γ-butyrolactone, γ-hydroxy butyric acid, δ-valerolactone, δ-hydroxy, valeric acid hydrooxybutyric acids, malic acid, and copolymers thereof. Other types of biodegradable polymers which can be used in the present invention are polyester amide, polyamide, polyether ester, poly anhydride, polyurethane, polyester urethane, polycarbonate and polyester carbonate. Calculating from the values for total molecular weight and percent by weight of A and B polymer blocks as given in Table 1, the average molecular weight of the polymeric A block is less than 10,000 daltons. By similar calculation, the hydrophilic B-block segment is polyethylene glycol (PEG) having an average molecular weight less than 5000.

The biodegradable diblock copolymers described may be synthesized by ring opening polymerization, or condensation polymerization. For example, the B(PEG) blocks may be coupled to the A blocks(polyesters) by ester or urethane links and the like. Condensation polymerization and ring opening polymerization procedures may be utilized as may the coupling of a monofunctional hydrophilic B block to either end of a di-functional hydrophobic A block in the presence of coupling agents such as isocyanates. Furthermore, coupling reactions may follow activation of functional groups with activating agents such as carbonyl diimidazole, succinic anhydride, maleic anhydride, glutaric anhydride, N-Hydroxy succinimide and p-nitrophenyl chloroformate and the like.

The hydrophilic B-block is formed from PEG of appropriate molecular weights. PEG was chosen as the hydrophilic, water-soluble block because of its unique biocompatibility, nontoxicity, hydrophilicity, solubilization properties, and rapid clearance from a patient's body.

The hydrophobic A-blocks are utilized because of their biodegradable, biocompatible, and solubilization properties. The *in vitro* and *in vivo* degradation of these hydrophobic, biodegradable polyester A-blocks is well understood and the degradation products are naturally occurring compounds that are readily metabolized and/or eliminated by the patient's body.

Surprisingly, the total weight percentage of the hydrophobic polyester A-block, relative to that of the hydrophilic PEG B-block, is high, e.g. between about 61 to 85% by weight, and is between about 65 to 78% by weight, yet the resulting diblock polymer retains the desired water solubility and reverse thermal gelation properties. It is an unexpected discovery that a block copolymer with such a large proportion of hydrophobic component would be water soluble below normal room temperature, such as refrigerator temperatures (5°C). It is believed that this desirable solubility characteristic is made possible by maintaining an overall low molecular weight of the entire diblock copolymer to between about 150 and 15000. Thus, water soluble biodegradable block copolymers possessing thermally reversible gelation properties are prepared wherein the hydrophilic B-block or blocks make up about 15 to 39% by weight of the copolymer and the hydrophobic A-block or blocks make up about 61 to 85% by weight of the copolymer. For example, the A-blocks (polyesters) may comprise between about 65 to 78% by weight of the copolymer and the PEG B-blocks may comprise between about 22 to 35% by weight of the copolymer. Furthermore, the preferred overall average molecular weight of the entire diblock copolymer will be between about 850 and 7000. The most preferred overall average molecular weight of the entire diblock copolymer will be between about 1000 and 5000.

The concentration at which the diblock copolymers are soluble at temperatures below the gelation temperature may be considered as the functional concentration. Generally speaking, diblock copolymer concentrations of as low as 3% and up to about 60% by weight can be used and still be functional. However, concentrations in the range of about 5 to 50% are preferred and concentrations in the range of about 25-45% by weight are most preferred. In order to obtain a viable gel phase transition with the copolymer, a certain minimum concentration, e.g. 20% by weight, is required. At the lower functional concentration ranges, phase transition may result in the formation of a weak gel. At higher concentrations, a strong gel network is formed.

The mixture of the biodegradable diblock copolymer and peptide/protein drugs, and/or other types of drugs, may be prepared as an aqueous solution of the diblock copolymer below the gelation temperature to form a drug delivery liquid wherein the drug may be either partially or completely dissolved. When the drug is partially dissolved, or when the drug is essentially insoluble, the drug exists in a colloidal state such as a suspension or emulsion. This drug delivery liquid is then administered parenterally, topically, transdermally, transmucosally, inhaled, or inserted into a cavity such as by ocular, vaginal, transurethral, rectal, nasal, oral, buccal, pulmonary or aural administration to a patient, whereupon it undergoes reversible thermal gelation since body temperature will be above the gelation temperature.

This system will cause minimal toxicity and minimal mechanical irritation to the surrounding tissue due to the biocompatibility of the materials and pliability of the gel, and will be completely biodegraded to lactic acid, glycolic acid, and other corresponding monomers within a specific time interval. The drug release, gel strength, gelation temperature and degradation rate can be controlled by proper design and preparation of the various copolymer blocks, namely, through modifications of the weight percent of A-blocks and B-blocks, the mole percentages of lactate and glycolate, and the molecular weight and polydispersity of the AB diblock copolymers. Drug release is also controllable through adjustment of the concentration of polymer in the drug delivery liquid.

A dosage form comprised of a solution of the diblock copolymer that contains either dissolved drug or drug as a suspension or emulsion is administered to the body. This formulation then spontaneously gels, due to the reverse thermal gelation properties of the block copolymer, to form a drug depot as the temperature of the formulation rises to body temperature. The only limitation as to how much drug can be loaded into the formulation is one of functionality, namely, the drug load may be increased until the thermal gelation properties of the copolymer are adversely affected to an unacceptable degree, or until the properties of the formulation are adversely affected to such a degree as to make administration of the formulation unacceptably difficult. Generally speaking, it is anticipated that in most instances the drug will make up between about 0.01 to 20% by weight of the formulation with ranges of between about 0.01 to 10% being most common. These ranges of drug loading are not limiting to the invention. Provided functionality is maintained, drug loadings oufside of these ranges fall within the scope of the invention.

A distinct advantage to the compositions of the subject of this invention lies in the ability of the diblock copolymer to increase the solubility of many drug substances. The combination of the hydrophobic A-block(s) and hydrophilic B-block(s) renders the diblock copolymer amphiphilic in nature. What is surprising is the degree of drug solubilization of most, if not all, drugs since the major component of the diblock copolymer is the hydrophobic A-block content. However, as already discussed, even though the hydrophobic polymer block(s) are the major component, the block copolymer is water soluble and it has been found that there is an additional increase in drug solubility when combined in an aqueous phase of the block copolymer.

Another advantage to the composition of the invention lies in the ability of the block copolymer to increase the chemical stability of many drug substances. Various mechanisms for the degradation of drugs, which lead to a drug's chemical instability, have been observed to be inhibited when the drug is in the presence of the diblock copolymers described. For example, paclitaxel and cyclosporin A are substantially stabilized in the aqueous polymer composition described relative to certain aqueous solutions of these same drugs in the presence of organic co-solvents. This stabilization effect on paclitaxel and cyclosporin A is but illustrative of the effect that can be achieved with many other drug substances.

In certain situations the drug loaded polymer may be administered in the gel state instead of as a solution. The gelation may be the result of raising the temperature of a drug laden polymer solution to above the gelation temperature of the polymer prior to administration, or may be caused by raising the concentration of the polymer in the solution to above the saturation concentration at the temperature of administration, or may be caused by addition of additives to the polymer solution which causes the solution to gel. In either event, the gel thus formed may be administered parenterally, topically, transdermally, transmucosally, inhaled or inserted into a cavity such as by ocular, vaginal, buccal, transurethral, rectal, nasal, oral, pulmonary or aural administration.

This invention is applicable to bioactive agents and drugs of all types including nucleic acids, hormones, anticancer-agents, and offers an unusually effective way to deliver polypeptides and proteins. Many labile peptide and protein drugs are amenable to formulation into the block copolymers of the invention and can benefit from the reverse thermal gelation process described herein. While not specifically limited to the following, examples of pharmaceutically useful polypeptides and proteins may be selected from the group consisting of erythropoietin, oxytocin, vasopressin, adrenocorticotropic hormone, epidermal growth factor, platelet-derived growth factor (PDGF), prolactin, luliberin, luteinizing hormone releasing hormone (LHRH), LHRH agonists, LHRH antagonists, growth hormone (human, porcine, bovine, etc.), growth hormone releasing factor, insulin, somatostatin, glucagon, interleukin-2 (IL-2), interferon-α,β, or γ, gastrin, tetragastrin, pentagastrin, urogastrone, secretin, calcitonin, enkephalins, endorphins, angiotensins, thyrotropin releasing hormone (TRH), tumor necrosis factor (TNF), nerve growth factor (NGF), granulocyte-colony stimulating factor (G-CSF), granulocyte macrophage-colony stimulating factor (GM-CSF), macrophage-colony stimulating factor (M-CSF), heparinase, bone morphogenic protein (BMP), hANP, glucagon-like peptide (GLP-1), interleukin-11 (IL-11), renin, bradykinin, bacitracins, polymyxins, colistins, tyrocidine, gramicidins, cyclosporins and synthetic analogues, modifications and pharmacologically active fragments thereof, enzymes, cytokines, antibodies and vaccines.

The only limitation to the polypeptide or protein drug which may be utilized is one of functionality. In some instances, the functionality or physical stability of polypeptides and proteins can also be increased by the addition of various additives to aqueous solutions or suspensions of the polypeptide or protein drug. Additives, such as polyols (including sugars), amino acids, surfactants, polymers, other proteins and certain salts may be used. These additives can be readily incorporated into the block copolymers which will then undergo the reverse thermal gelation process described.

Developments in protein engineering may provide the possibility of increasing the inherent stability of peptides or proteins. While such resultant engineered or modified proteins may be regarded as new entities in regards to regulatory implications, this does not alter their suitability for use in the present invention. One of the typical examples of modification is PEGylation wherein the stability of the polypeptide drugs can be significantly improved by covalently conjugating water-soluble polymers, such as polyethylene glycol, with the polypeptide. Another example is the modification of the amino acid sequence in terms of the identity or location of one or more amino acid residues by terminal and/or internal addition, deletion or substitution. Any improvement in stability enables a therapeutically effective polypeptide or protein to be continuously released over a prolonged period of time following a single administration of the drug delivery liquid to a patent.

In addition to peptide or protein based drugs, other drugs from all therapeutic and medically useful categories may be utilized. These drugs are described in such well-known literature references as the Merck Index, the Physicians Desk Reference, and The Pharmacological Basis of Therapeutics. A brief listing of specific agents is described for illustration purposes only, and shall not be deemed as limiting: anticancer agents such as mitomycin, bleomycin, BCNU, carboplatin, doxorubicin, daunorubicin, methotrexate, paclitaxel, taxotere, actinomycin D and camptothecin; antipsychotics such as olanzapine and ziprasidone; antibacterials such as cefoxitin; anthelmintics such as ivermectin; antivirals such as acyclovir; immunosuppressants such as cyclosporin A (cyclic polypeptide-type agent), steroids, and prostaglandins.

### Example 1

### Synthesis of MeO-PEG-PLGA Diblock Copolymer by Ring Opening; Copolymerization

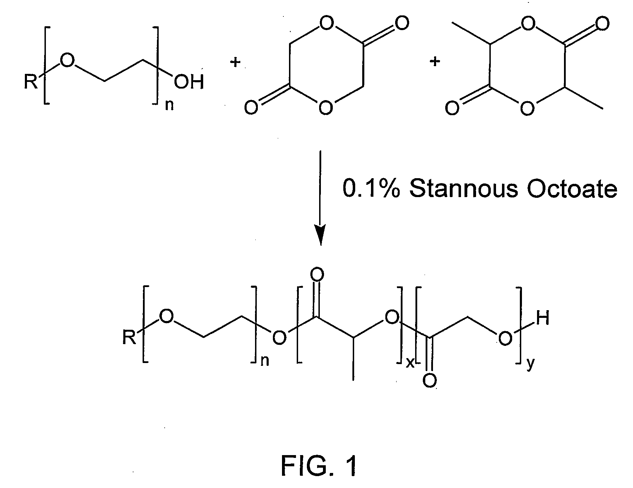

Following the reaction scheme given in FIG. 1, 50 g of MeO-PEG (Mw= 550) was dried for 2 hrs at 100°C under vacuum (5 mm Hg). 89.58 g of D,L=lactide and 28.15 g of glycolide were added to the flask and the reaction mixture was heated until the temperature reached 155°C. When the temperature of reaction mixture was 130°C, 0.04 g (700 ppm) of stannous octoate was added into the reaction flask using a 1 mL syringe. The progress of the reaction was followed by GPC (gel permeation chromatography). When the molecular weight of the copolymer showed no further increase, the reaction was stopped and the flask was cooled and any unreacted monomers were removed by vacuum distillation for 2 hrs.

### Example 2

Following the general procedure outlined in Example 1, other diblock copolymers were synthesized using the same PEG but varying the lactide and/or glycolide content. The properties of these diblock copolymers are listed in the following table:

**Table 1. Example AB Diblock Copolymers with Reverse Thermal Gelation Properties**

| PEG-OMe | PLG/PEG | L/G | Mₙ(calculated) | Mₙ (GPC) | T_{sol-gel} | T_{max visc} | T_{ph sepa} |
|---|---|---|---|---|---|---|---|
| 550 | 2.58 | 75/25 | 1969 | 2390 | 17.4 | 19.7-39.6 | 42.8 |
| 550 | 2.80 | 75/25 | 2090 | 2450 | 20.8 | 24.4-49.1 | 51.5 |
| 550 | 3.0 | 72/28 | 2200 | 2630 | 23.1 | 25.1-53.9 | 58.1 |
| 750 | 2.6 | 75/25 | 2700 | 3180 | 38.2 | 43.1-52.8 | 56.0 |
| 750 | 3.0 | 75/25 | 3000 | 3510 | 52.3 | 55.1-71.3 | >77.0 |

It is to be noted that all of the polymers listed in the above table possessed reverse thermal gelation properties and the diblock copolymer concentrations for gelation properties were 23 wt% or 25 wt% in water. Molecular weight and gelation properties of diblock copolymer solutions have been characterized both by gel permeation chromatography (GPC) and the tube inversion method.

### Example 3

### Synthesis of PLGA-PEG Diblock Copolymer by Condensation Copolymerization.

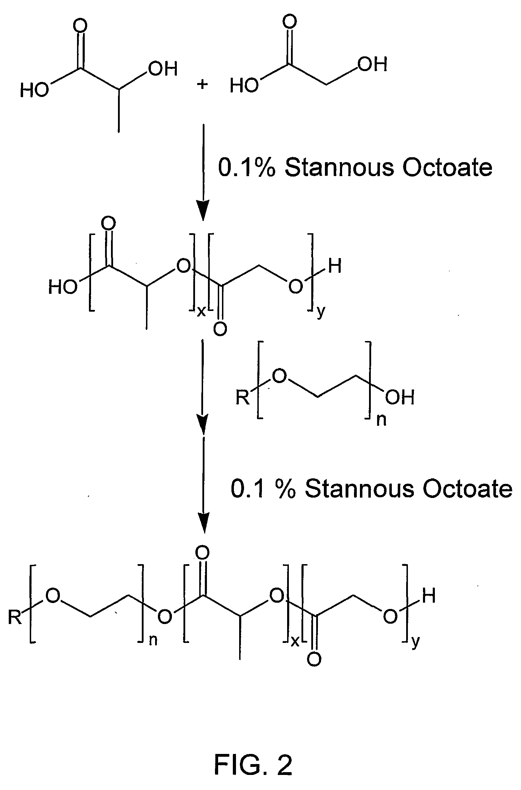

The reaction scheme of diblock copolymer synthesis by condensation polymerization is presented in FIG. 2. Into a three necked flask, equipped with a nitrogen inlet, thermometer, and distillation head for removal of water, was placed DL-lactic acid and glycolic acid (3:1 mole ratio, respectively). The reaction mixture was heated at 155°C under nitrogen, with stirring, at atmospheric pressure for three hours and then under reduced pressure (5mm Hg) for 6 hrs. The progress of the reaction was followed by GPC. The reaction was stopped at the appropriate time and the polymer formed was purified by precipitation from a dichloromethane solution into a large excess of methanol. The residue was triturated with methanol and dried under vacuum (0.05 mm Hg) at 23°C. The PLGA oligomer was characterized by GPC, IR and NMR. The resulting PLGA oligomer had a number average molecular weight (Mn) of 1910 and an Mw/Mn ratio of 1.8.

The PLGA was mixed with MeO- PEG (Mw=550) and heated in a flask at 160°C under a nitrogen atmosphere. A 0.1 wt % of stannous octoate was used as catalyst. The progress of the reaction was followed by GPC. After an appropriate time, the reaction was stopped and the flask was cooled to room temperature. The resulting PLGA-PEG diblock copolymer had a number average molecular weight (Mn) of 2260 and an Mw/Mn ratio of 1.6. The weight average molecular weights and number average molecular weights were determined by GPC and NMR, respectively. The lactide/glycolide ratio was calculated from NMR data. GPC analysis was performed on a Styragel HR-3 column calibrated with PEG using RI detection and chloroform as the eluent. NMR spectra were taken in CDCl₃ on a Bruker 200 MHZ instrument. NMR peak assignments confirmed the diblock AB structure.

### Example 4

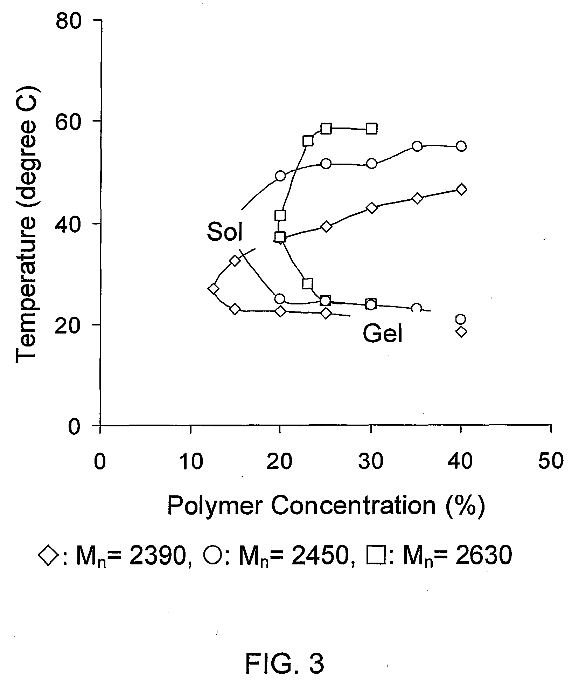

The gelation behavior of aqueous solutions of various AB diblock copolymers described was studied at different concentrations. Polymer solutions of 10-40% by weight were prepared in water and the change in viscosity was observed at temperatures ranging between 10°C and 60°C. Gelation was defined as the physical state where the polymer solution did not readily flow upon inverting a vial of polymer solution. The phase diagram (FIG. 3) of the polymer of Example 2 as a function of temperature and diblock copolymer concentration was generated from the gelation study. The novel, reverse thermal gelation behavior was clearly apparent, and occurred as the diblock copolymer solutions were heated. The gelation at physiologically relevant temperatures (e.g., 37°C) was particularly prevalent and formed the basis for the substantial utility of the systems for medical and drug delivery purposes.

### Example 5

The *in vitro* degradation of the PLG-PEG diblock copolymer of Example 1 is determined for a 25% by weight solution or gel (1 ml) of copolymer incubated at different temperatures (- 10°C, 5°C , 23°C , and 37°C) and at different initial pH's (3.0, 5.0 and 7.4) over a 12 week period. The degradation and biodegradation of this diblock copolymer is caused by hydrolysis and results in lactic acid, glycolic acid and PEG as the final degradation products.

Samples (50 µl) are taken weekly. The samples were lyophilized, dissolved in chloroform, and the molecular weights of the polymer residues are determined by GPC as described previously. The degradation of the polymer is substantially independent of initial pH over a range of pHs from 3.0 to 7.4, which can be attributed to acidification of the media as the polymer hydrolyzed to form lactic acid and glycolic acid. The thermal gelling behavior is also independent of the pH over the same pH range. The degradation is more rapid at higher temperatures.

### Example 6

The *in vivo* biodegradation of the diblock copolymer of Example 1 is determined over an eight week period. A 0.40 to 0.45 ml sample of a cold aqueous solution containing 23% by weight diblock copolymer is injected subcutaneously into rats. Upon reaching body temperature, which is above the gelation temperature of the polymer, a gel lump immediately forms which is visibly apparent. Samples are surgically retrieved and indicated that the gel became progressively smaller as a function of time over a four week period. Between four weeks and eight weeks, the physical state of the injected diblock copolymer changes from a gel to a mixture of a gel in a viscous liquid, and finally to a viscous liquid containing no gel. This liquid is gradually completely resorbed. At the end of the eight week period, no formulation is visible at the injection site. Microscopically, small pockets of viscous liquid are observable that also resorbed completely over the following two week period.

### Example 7

Paclitaxel and cyclosporin A are hydrophobic drugs that are highly insoluble in water (solubilities were approximately 4 µg/ml). However, these drugs show significantly higher solubilities when dissolved in aqueous solutions of PLG-PEG diblock copolymers. For example, in a 30 % by weight aqueous copolymer solution (polymer of Example 1), paclitaxel is soluble up to 6 mg/ml and cyclosporin A is soluble up to 2 mg/ml.

Paclitaxel and cyclosporin A are highly unstable in aqueous cosolvent solutions (e.g. in water/acetonitrile solutions). The paclitaxel contained in either 30 % by weight aqueous PLG-PEG diblock copolymer solutions (i.e., below the gelation temperature of the copolymer) or gels (i.e., above the gelation temperature of the copolymer) is greater than 85% intact after 120 days in storage (5°C and 37°C), whereas cyclosporin A is stable over 100 days (5°C).

### Example 8

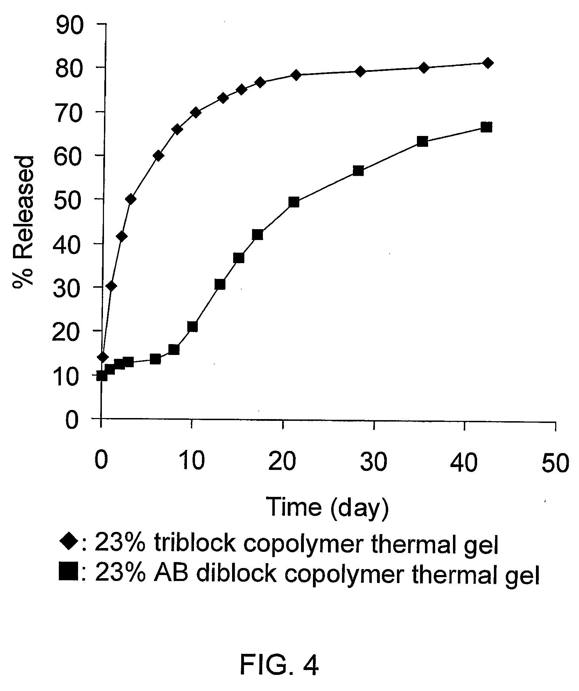

Various concentrations by weight of aqueous solutions of the PLG-PEG diblock copolymer with a number average molecular weight of 2390 in Table 1 were prepared. FITC dextran of molecular weights 40,000 and 4000 were dissolved in this aqueous solution of diblock copolymers to give a final concentration of 5 mg/mL. One gram of polymer sample was loaded in a glass scintillation vial. After incubation at 37°C for 15 min to form a firm gel, 10 mL of 10 mM PBS was added to the vial. As time passed, the medium was replaced with fresh medium. FITC dextran released media was analyzed by a UV/VIS spectrometer at 493 nm. Throughout the study, the polymer solutions were kept in an incubator in which the temperature was set to 37°C. Samples were agitated by a shaker at 75 rpm. The data has been graphically summarized in FIG. 4. FITC dextran 40000 was released from the diblock copolymer gel in a sustained manner. At a concentration of 23 wt% of the diblock copolymer, about 60 % of the incorporated FITC dextran was released in 42 days. However, a triblock copolymer (ABA structure) which has been used as positive control showed faster release of FITC dextran than the diblock copolymer (70 % in 10 days).

As illustrated in Fit.5, sustained release of FITC dextran from diblock copolymer thermal gels has been further noted by increases in diblock copolymer concentrations. As the concentration is increased from 30 to 40 wt%, FITC dextran 4000 release is sustained (30 % and 24 % in 10 days for 30 wt% and 40 wt%, respectively). Notably, there was no significant burst in initial 1 day release(less than 10 %). As the polymer concentration increased from 30 to 40 %, the initial burst release of FITC dextran 4,000 within 4 hours was significantly reduced from 5.6 % to 3.0 % (FIG 5-a). Compared to a triblock copolymer thermal gel, the diblock copolymer gel showed a noticeable reduction in initial burst. At a polymer concentration of 30 %, the initial burst release of FITC dextran 4,000 from a triblock copolymer (BAB structure) gel was 24 % while release from a diblock copolymer gel released was only 6% in 4 hours (FIG 5-b).

The release profile of FITC dextran has also been modulated by mixing two different diblock copolymers (FIG. 5-c). A mixture of two different diblock copolymers of which gelation temperatures are different shows different release kinetics of FITC dextran 4,000. With the mixing of diblock copolymers with higher gelation temperature, release of FITC dextran from gel matrix is been facilitated. This demonstration clearly indicates that mixing of two different diblock copolymers with different gel properties could be useful for modulation of release kinetics and gelation temperature.

Therefore, sustained drug release from diblock copolymer thermal gel for a substantial period was clearly established and illustrated by this Example.

The above description will enable one skilled in the art to make AB type diblock copolymers that form aqueous solutions having reverse thermal gelation properties and to utilize the same in the field of drug delivery. Although the controlled delivery of FITC dextran of molecular weights 40,000 and 4000 are illustrated in the examples to show the functionality of hydrogels formed from aqueous solutions of diblock copolymers, these descriptions are not intended to be an exhaustive statement of all drugs which can be utilized and loaded into the biodegradable block copolymers. Certainty, numerous other drugs from various classes of therapeutic agents are well suited for delivery from aqueous compositions of diblock copolymers as described in this description of the invention. Neither are all diblock copolymers which may be prepared, and which demonstrate the critical reverse thermal gelation property, specifically shown. However, it will be immediately apparent to one skilled in the art that various modifications may be made without departing from the scope of the invention which is limited only by the following claims and their functional equivalents.

## Claims

1. An aqueous biodegradable polymeric drug delivery composition possessing reverse thermal gelation properties comprised of an aqueous phase having uniformly contained therein:
(a) an effective amount of a drug; and
(b) a biodegradable AB type di block copolymer comprising:
i) 61 to 85 % by weight of a biodegradable, hydrophobic A block which is a member selected from the group consisting of biodegradable polyesters, biodegradable polyester amides, biodegradable polyether esters, biodegradable polyurethanes, biodegradable polyester urethanes, biodegradable polycarbonates and polyester carbonates; and
ii) 15 to 39 % by weight of a biocompatible, hydrophilic B block comprising a mono functional polyethylene glycol(PEG),
wherein, each hydrophobic A block has a number average molecular weight of between 1000 Daltons and 4000 Daltons and each hydrophilic B block has a number average molecular weight of between 500 Daltons and 800 Daltons; and
wherein said di block copolymer has a number average molecular weight within a range of 450 Daltons to 15000 Daltons and
wherein the copolymer possesses reverse thermal gelation properties such that it exists as a solution at about 5-34°C in water, but at 37°C, the copolymers spontaneously interact to form a semisolid hydrogel.

2. The aqueous polymeric composition according to Claim 1, wherein the biodegradable, hydrophobic A block is synthesized from monomers selected from the group consisting of D,L-lactide , D-lactide, L-lactide, D,L-lactic acid, D-lactic acid, L-lactic acid, glycolide, glycolic acid, p-dioxanone, trimethylene carbonate, ε-caprolactone, ε-hydroxy hexonoic acid, γ-butyrolactone, γ-hydroxy butyric acid, δ-valerolactone, δ-hydroxy valeric acid, hydrooxybutyric acids, malic acid, and copolymers thereof.

3. The aqueous polymeric composition according to claims 1 or 2 wherein the di block polymer content of said composition is between 3 and 50% by weight and the drug content of said composition is between 0.01 and 20% by weight.

4. A method for enhancing the solubility of a drug, comprising uniformly admixing an effective amount of said drug in an aqueous biodegradable polymeric drug delivery composition possessing reverse thermal gelation properties, said aqueous composition being comprised of an aqueous phase having uniformly contained therein a biodegradable AB type di block copolymer comprising:
i) 61 to 85 % by weight of a biodegradable, hydrophobic A block which is a member selected from the group consisting of biodegradable polyesters, biodegradable polyester amides, biodegradable polyether esters, biodegradable polyurethanes, biodegradable polyester urethanes, biodegradable polycarbonates and polyester carbonates; and
ii) 15 to 39 % by weight of a biocompatible, hydrophilic B block comprising a mono functional polyethylene glycol(PEG),
wherein, each hydrophobic A block has a number average molecular weight of between 1000 Daltons and 4000 Daltons and each hydrophilic B block has a number average molecular weight of between 500 Daltons and 800 Daltons; and
wherein the copolymer possesses reverse thermal gelation properties such that it exists as a solution at about 5-34°C in water, but at 37°C, the copolymers spontaneously interact to form a semisolid hydrogel.

5. A method for enhancing the stability of a drug comprising uniformly admixing an effective amount of said drug in an aqueous biodegradable polymeric drug delivery composition possessing reverse thermal gelation properties said aqueous composition being comprised of an aqueous phase having uniformly contained therein a biodegradable AB type di block copolymer comprising:
i) 61 to 85 % by weight of a biodegradable, hydrophobic A block which is a member selected from the group consisting of biodegradable polyesters, biodegradable polyester amides, biodegradable polyether esters, biodegradable polyurethanes, biodegradable polyester urethanes, biodegradable polycarbonates and polyester carbonates; and
ii) 15 to 39 % by weight of a biocompatible, hydrophilic B block comprising a mono functional polyethylene glycol(PEG),
wherein, each hydrophobic A block has a number average molecular weight of between 1000 Daltons and 4000 Daltons and each hydrophilic B block has a number average molecular weight of between 500 Daltons and 800 Daltons; and
wherein the copolymer possesses reverse thermal gelation properties such that it exists as a solution at about 5-34°C in water, but at 37°C, the copolymers spontaneously interact to form a semisolid hydrogel.

6. The method according to Claim 4 or Claim 5, wherein the biodegradable, hydrophobic A block is synthesized from monomers selected from the group consisting of D,L-lactide , D-lactide, L-lactide, D,L-lactic acid, D-lactic acid, L-lactic acid, glycolide, glycolic acid, p-dioxanone, trimethylene carbonate, ε-caprolactone, ε-hydroxy hexonoic acid, γ-butyrolactone, γ-hydroxy butyric acid, δ-vaterolactone, δ-hydroxy valeric acid, hydrooxybutyric acids, malic acid, and copolymers thereof.

7. The method according to Claim 4 or Claim 5, wherein the di block polymer content of said composition is between 3 and 50% by weight and the drug content of said composition is between 0.01 and 20% by weight.

## Patentansprüche

1. Wässrige, biologisch abbaubare Polymerzusammensetzung zur Arzneimittelzuführung, die reversible thermische Gelbildungseigenschaften besitzt und die eine wässrige Phase aufweist, welche darin gleichförmig enthält:
(a) eine wirksame Menge eines Arzneimittels und
(b) ein biologisch abbaubares Diblockcopolymer vom AB-Typ, umfassend:
i) 61% bis 85 Gew.-% eines biologisch abbaubaren, hydrophoben A-Blocks, der ein Vertreter ist, ausgewählt aus der Gruppe, bestehend aus biologisch abbaubaren Polyestern, biologisch abbaubaren Polyesteramiden, biologisch abbaubaren Polyetherestern, biologisch abbaubaren Polyurethanen, biologisch abbaubaren Polyesterurethanen, biologisch abbaubaren Polycarbonaten, biologisch abbaubaren Polyestercarbonaten; und
ii) 15% bis 39 Ges.-% eines biokompatiblen hydrophilen B-Blocks, umfassend ein monofunktionelles Polyethylenglykol (PEG),
wobei jeder hydrophobe A-Block ein Zahlenmittel des Molekulargewichts zwischen 1.000 Dalton und 4.000 Dalton hat und jeder hydrophile B-Block ein Zahlenmittel des Molekulargewichts zwischen 500 Dalton und 800 Dalton hat, und wobei das Diblockcopolymer ein Zahlenmittel des Molekulargewichts im Bereich von 450 Dalton bis 15.000 Dalton hat und wobei das Copolymer reversible thermische Gelbildungseigenschaften besitzt, so dass es in Wasser bei etwa 5° bis 34 °C als eine Lösung existiert, die Copolymere jedoch bei 37 °C spontan wechselwirken, um halbfeste Hydrogele zu bilden.

2. Wässrige Polymerzusammensetzung nach Anspruch 1, wobei der biologisch abbaubaren hydrophobe A-Block synthetisch dargestellt wird aus Monomeren, die ausgewählt sind aus der Gruppe, bestehend aus D,L-Lactid, D-Lactid, L-Lactid, D,L-Milchsäure, D-Milchsäure, L-Milchsäure, Glykolid, Glykolsäure, p-Dioxanon, Trimethylencarbonat, ε-Caprolacton, ε-Hydroxyhexansäure, γ-Butyrolacton, γ-Hydroxybutansäure, δ-Valerolacton, δ-Hydroxypentansäure, Hydrooxybutansäuren, Hydroxybernsteinsäure und Copolymere davon.

3. Wässrige Polymerzusammensetzung nach Anspruch 1 oder 2, wobei der Gehalt an Diblockpolymer der Zusammensetzung zwischen 3% und 50 Gew.-% beträgt und der Arzneimittelgehalt der Zusammensetzung zwischen 0,01% und 20 Gew.-% beträgt.

4. Verfahren zum Erhöhen der Löslichkeit eines Arzneimittels, umfassend das gleichmäßige Zumischen einer wirksamen Menge des Arzneimittels in einer wässrigen, biologisch abbaubaren Polymerzusammensetzung zur Arzneimittelzuführung, die reversible thermische Gelbildungseigenschaften besitzt und die eine wässrige Phase aufweist, welche darin gleichförmig enthält:
(a) eine wirksame Menge eines Arzneimittels und
(b) ein biologisch abbaubares Diblockcopolymer vom AB-Typ, umfassend:
i) 61% bis 85 Gew.-% eines biologisch abbaubaren, hydrophoben A-Blocks, der ein Vertreter ist, ausgewählt aus der Gruppe, bestehend aus biologisch abbaubaren Polyestern, biologisch abbaubaren Polyesteramiden, biologisch abbaubaren Polyetherestern, biologisch abbaubaren Polyurethanen, biologisch abbaubaren Polyesterurethanen, biologisch abbaubaren Polycarbonaten, biologisch abbaubaren Polyestercarbonaten; und
ii) 15% bis 39 Gew.-% eines biokompatiblen hydrophilen B-Blocks, umfassend ein monofunktionelles Polyethylenglykol (PEG),
wobei jeder hydrophobe A-Block ein Zahlenmittel des Molekulargewichts zwischen 1.000 Dalton und 4.000 Dalton hat und jeder hydrophile B-Block ein Zahlenmittel des Molekulargewichts zwischen 500 Dalton und 800 Dalton hat, und wobei das Diblockcopolymer ein Zahlenmittel des Molekulargewichts im Bereich von 450 Dalton bis 15.000 Dalton hat und wobei das Copolymer reversible thermische Gelbildungseigenschaften besitzt, so dass es in Wasser bei etwa 5° bis 34 °C als eine Lösung existiert, die Copolymere jedoch bei 37 °C spontan wechselwirken, um halbfeste Hydrogele zu bilden.

5. Verfahren zum Erhöhen der Stabilität eines Arzneimittels, umfassend das gleichmäßige Zumischen einer wirksamen Menge des Arzneimittels in einer wässrigen, biologisch abbaubaren Polymerzusammensetzung zur Arzneimittelzuführung, die reversible thermische Gelbildungseigenschaften besitzt und die eine wässrige Phase aufweist, welche darin gleichförmig enthält:
(a) eine wirksame Menge eines Arzneimittels und
(b) ein biologisch abbaubares Diblockcopolymer vom AB-Typ, umfassend:
i) 61% bis 85 Gew.-% eines biologisch abbaubaren, hydrophoben A-Blocks, der ein Vertreter ist, ausgewählt aus der Gruppe, bestehend aus biologisch abbaubaren Polyestern, biologisch abbaubaren Polyesteramiden, biologisch abbaubaren Polyetherestern, biologisch abbaubaren Polyurethanen, biologisch abbaubaren Polyesterurethanen, biologisch abbaubaren Polycarbonaten, biologisch abbaubaren Polyestercarbonaten; und
ii) 15% bis 39 Gew.-% eines biokompatiblen hydrophilen B-Blocks, umfassend ein monofunktionelles Polyethylenglykol (PEG),
wobei jeder hydrophobe A-Block ein Zahlenmittel des Molekulargewichts zwischen 1.000 Dalton und 4.000 Dalton hat und jeder hydrophile B-Block ein Zahlenmittel des Molekulargewichts zwischen 500 Dalton und 800 Dalton hat, und wobei das Diblockcopolymer ein Zahlenmittel des Molekulargewichts im Bereich von 450 Dalton bis 15.000 Dalton hat und wobei das Copolymer reversible thermische Gelbildungseigenschaften besitzt, so dass es in Wasser bei etwa 5° bis 34 °C als eine Lösung existiert, die Copolymere jedoch bei 37 °C spontan wechselwirken, um halbfeste Hydrogele zu bilden.

6. Verfahren nach Anspruch 4 oder 5, wobei der biologisch abbaubaren hydrophobe A-Block synthetisch dargestellt wird aus Monomeren, die ausgewählt sind aus der Gruppe, bestehend aus D,L-Lactid, D-Lactid, L-Lactid, D,L-Milchsäure, D-Milchsäure, L-Milchsäure, Glykolid, Glykolsäure, p-Dioxanon, Trimethylencarbonat, ε-Caprolacton, ε-Hydroxyhexansäure, γ-Butyrolacton, γ-Hydroxybutansäure, δ-Valerolacton, δ-Hydroxypentansäure, Hydrooxybutansäuren, Hydroxybernsteinsäure und Copolymere davon.

7. Verfahren nach Anspruch 4 oder 5, wobei der Gehalt an Diblockpolymer der Zusammensetzung zwischen 3% und 50 Gew.-% beträgt und der Arzneimittelgehalt der Zusammensetzung zwischen 0,01% und 20 Gew.-% beträgt.

## Revendications

1. Composition aqueuse polymère biodégradable d'administration de médicament possédant des propriétés de gélification thermique inverse constituée d'une phase aqueuse qui contient uniformément :
(a) une quantité efficace d'un médicament; et
(b) un copolymère à deux séquences de type AB biodégradable comprenant :
i) 61 à 85 % en poids d'une séquence A hydrophobe, biodégradable qui est un membre choisi dans le groupe constitué de polyesters biodégradables, polyesteramides biodégradables, polyétheresters biodégradables, polyuréthanes biodégradables, uréthanes de polyesters biodégradables, polycarbonates biodégradables et carbonates de polyester ; et
ii) 15 à 39 % en poids d'une séquence B hydrophile, biocompatible comprenant un polyéthylène glycol (PEG) mono fonctionnel,
dans lequel chaque séquence A hydrophobe a un poids moléculaire moyen en nombre compris entre 1000 et 4000 Daltons et chaque séquence B hydrophile a un poids moléculaire moyen en nombre compris entre 500 et 800 Daltons ; et dans lequel ledit copolymère à deux séquences a une masse moléculaire moyenne en nombre dans la plage de 450 à 15000 Daltons, et
dans lequel le copolymère possède des propriétés de gélification thermique inverse de telle sorte qu'il existe sous forme d'une solution à environ 5 à 34 °C dans l'eau, mais à 37 °C, les copolymères interagissent spontanément pour former un hydrogel semi-solide.

2. Composition polymère aqueuse selon la revendication 1, dans lequel la séquence A hydrophobe, biodégradable est synthétisée à partir de monomères choisis dans le groupe constitué de DL-lactide, D-lactide, L-lactide, acide DL-lactique, acide D-lactique, acide L-lactique, glycolide, acide glycolique, p-dioxanone, carbonate de triméthylène, ε-caprolactone, acide ε-hydroxy hexonoïque, γ-butyrolactone, acide γ-hydroxy butyrique, δ-valérolactone, l'acide δ-hydroxy valérique, acides hydrooxybutyriques, acide malique et leurs copolymères.

3. Composition polymère aqueuse selon la revendication 1 ou 2, dans laquelle la teneur en polymère à deux séquence de ladite composition est comprise entre 3 et 50 % en poids et la teneur en médicament de ladite composition est comprise entre 0,01 et 20 % en poids.

4. Procédé pour améliorer la solubilité d'un médicament, comprenant le mélange uniforme d'une quantité efficace dudit médicament dans une composition aqueuse polymère biodégradable d'administration de médicament possédant des propriétés de gélification thermique inverse, ladite composition aqueuse étant constituée d'une phase aqueuse contenant uniformément un copolymère à deux séquences de type AB biodégradable comprenant :
i) 61 à 85 % en poids d'une séquence A hydrophobe, biodégradable qui est un membre choisi dans le groupe constitué de polyesters biodégradables, polyesteramides biodégradables, polyétheresters biodégradables, polyuréthanes biodégradables, uréthanes de polyesters biodégradables, polycarbonates biodégradables et carbonates de polyester ; et
ii) 15 à 39 % en poids d'une séquence B hydrophile, biocompatible comprenant un polyéthylène glycol (PEG) mono fonctionnel,
dans lequel chaque séquence A hydrophobe a un poids moléculaire moyen en nombre compris entre 1000 et 4000 Daltons et chaque séquence B hydrophile a un nombre de poids moléculaire moyen compris entre 500 et 800 Daltons ; et
dans lequel le copolymère possède les propriétés de gélification thermique inverse de telle sorte qu'il existe sous la forme d'une solution à environ 5 à 34 °C dans l'eau, mais à 37 °C, les copolymères interagissent spontanément pour former un hydrogel semi-solide.

5. Procédé pour améliorer la stabilité d'un médicament, comprenant un mélange uniforme d'une quantité efficace dudit médicament dans une composition aqueuse polymère biodégradable d'administration de médicament possédant des propriétés de gélification thermique inverse, ladite composition aqueuse étant constituée d'une phase aqueuse contenant uniformément un copolymère à deux séquences de type AB biodégradable comprenant :
i) 61 à 85 % en poids d'une séquence A hydrophobe, biodégradable qui est un membre choisi dans le groupe constitué de polyesters biodégradables, polyesteramides biodégradables, polyétheresters biodégradables, polyuréthanes biodégradables, uréthanes de polyesters biodégradables, polycarbonates biodégradables et carbonates de polyester ; et
ii) 15 à 39 % en poids d'une séquence B hydrophile, biocompatible comprenant un polyéthylène glycol (PEG) mono fonctionnel,
dans lequel chaque séquence A hydrophobe a un poids moléculaire moyen en nombre compris entre 1000 et 4000 Daltons et chaque séquence B hydrophile a un nombre de poids moléculaire moyen compris entre 500 et 800 Daltons ; et
dans lequel le copolymère possède les propriétés de gélification thermique inverse de telle sorte qu'il existe sous la forme d'une solution à environ 5 à 34 °C dans l'eau, mais à 37 °C, les copolymères interagissent spontanément pour former un hydrogel semi-solide.

6. Procédé selon la revendication 4 ou 5, dans lequel la séquence A biodégradable, hydrophobe est synthétisée à partir de monomères choisis dans le groupe constitué de DL-lactide, D-lactide, L-lactide, acide DL-lactique, acide D-lactique, acide L-lactique, glycolide, acide glycolique, p-dioxanone, carbonate de triméthylène, ε-caprolactone, acide ε-hydroxy hexonoïque, γ-butyrolactone, acide γ-hydroxy butyrique, δ-valérolactone, l'acide δ-hydroxy valérique, acides hydrooxybutyriques, acide malique, et leurs copolymères.

7. Procédé selon la revendication 4 ou 5, dans lequel la teneur en polymère à deux séquences de ladite composition est comprise entre 3 et 50 % en poids, et la teneur en médicament de ladite composition est comprise entre 0,01 et 20 % en poids.
